# EUROPÄISCHE PATENTSCHRIFT

(11) **EP 0 889 944 B1**
(45) Veröffentlichungstag und Bekanntmachung des Hinweises auf die Patenterteilung: **22.12.1999**
(21) Anmeldenummer: 97914318.7
(22) Anmeldetag: 27.03.1997
(51) Int. Cl.: C11B 5/00, C08B 37/00, A61K 7/00, A61K 47/48

(54) **VERFAHREN ZUR STABILISIERUNG UND DISPERGIERUNG VON PFLANZENÖLEN, DIE MEHRFACH UNGESÄTTIGTE FETTSÄURERESTE ENTHALTEN, MITTELS gamma-CYCLODEXTRIN**
PROCESS FOR STABILISING AND DISPERSING VEGETABLE OILS WHICH CONTAIN POLYUNSATURATED FATTY ACID RADICALS BY MEANS OFgamma-CYCLODEXTRIN
PROCEDE DE STABILISATION ET DE DISPERSION D'HUILES VEGETALES QUI CONTIENNENT DES RESIDUS D'ACIDES GRAS POLYINSATURES AU MOYEN DE gamma-CYCLODEXTRINE

(30) Priorität: 29.03.1996 DE 19612658
(43) Veröffentlichungstag der Anmeldung: 13.01.1999
(73) Patentinhaber: WACKER-CHEMIE GMBH, D-81737 München (DE)
(72) Erfinder: WIMMER, Thomas, D-84533 Marktl (DE); REGIERT, Marlies, D-80538 München (DE); MOLDENHAUER, Jens-Peter, D-84489 Burghausen (DE)
(74) Vertreter: Potten, Holger
(86) Internationale Anmeldenummer: EP9701581
(87) Internationale Veröffentlichungsnummer: WO9736972

(56) Entgegenhaltungen:
- EP-A- 0 392 608
- EP-A- 0 470 452
- FR-A- 2 596 617
- US-A- 4 533 637
- US-A- 4 803 077
- JOURNAL OF INCLUSION PHENOMENA AND MOLECULAR RECOGNITION IN CHEMISTRY, Bd. 25, Nr. 1, 1996, DORDRECHT, NL, Seiten 213-216, XP002036271 M. REGIERT ET AL.: "Application of gamma-cyclodextrin for the stabilization and/or dispersion of vegetable oils containing triglycerides of polyunsaturated acids"
- DATABASE WPI Section Ch, Week 9541 Derwent Publications Ltd., London, GB; Class B04, AN 95-317436 XP002036274 & JP 07 215 911 A (ENSUIKO SUGAR REFINING CO LTD) , 15.August 1995 & PATENT ABSTRACTS OF JAPAN vol. 95, no. 11, 26.Dezember 1995 & JP 07 215911 A (ENSUIKO SUGAR REFINING CO LTD), 15.August 1995,
- YUKAGAKU - JOURNAL OF THE JAPAN OIL CHEMISTS' SOCIETY, Bd. 41, Nr. 3, 1992, JP, Seiten 203-206, XP002036272 K. ASAKURA ET L. : "Affinity of cyclodextrins to hydroperoxides"
- COLLOIDS AND SURFACES A, Bd. 97, Nr. 3, 1995, AMSTERDAM, NL, Seiten 263-269, XP002036273 R. BRU ET AL.: "Aggregation of polyunsaturated fatty acids in the presence of cycldextrins"
- DATABASE WPI Section Ch, Week 8751 Derwent Publications Ltd., London, GB; Class B04, AN 87-359752 XP002036275 & JP 62 263 143 A (KAO CORP) , 16.November 1987

## Beschreibung

Die Erfindung betrifft Verfahren zur Stabilisierung und Dispergierung von Pflanzenölen, die mehrfach ungesättigte Fettsäurereste enthalten mittels γ-Cyclodextrin.

Cyclodextrine sind cyclische Oligosaccharide, die aus 6,7 oder 8 α(1-4)-verknüpften Anhydroglukoseeinheiten aufgebaut sind. Die beispielsweise durch enzymatische Stärkekonversion hergestellten α-, β- oder γ-Cyclodextrine unterscheiden sich in dem Durchmesser ihrer hydrophoben Kavität und eignen sich generell zum Einschluß zahlreicher lipophiler Substanzen.

Pflanzenöle bestehen überwiegend aus einfachen und gemischten Triacylglycerinen, aufgebaut aus mit Glycerin veresterten, gesättigten, einfach- oder mehrfach ungesättigten natürlichen Fettsäureresten mit Kettenlängen kleiner gleich 18 Kohlenstoffatome.

Pflanzenöle mit einem hohen Anteil an Triacylglycerinen enthaltend mehrfach ungesättigte Fettsäuren sind wertvolle Substanzen, deren hautpflegende Eigenschaften in der Kosmetik genutzt werden (skin care). Darüberhinaus werden sie im Food-Bereich zur Versorgung mit essentiellen Fettsäuren eingesetzt.

Das Hauptproblem für die breitere Anwendung dieser Öle besteht in ihrer Empfindlichkeit gegen Luftsauerstoff, Wärme und Mikroorganismen, insbesondere unter Lichteinwirkung, wobei Peroxide gebildet werden (Autoxidation des ungesättigten Fettsäurerestes). An der Stelle der C-C Doppelbindung findet eine Autoxidation statt, die primär zur Bildung von Peroxiden, dann zu Aldehyden, Ketonen und Säuren führt. In Sekundär-Reaktionen treten Isomerisierungen und Polymerisationen ein.

Der Peroxidgehalt, ausgedrückt durch die Peroxidzahl (POZ), stellt das entscheidende Qualitätskriterium für Pflanzenöle dar. Öle mit hoher Peroxidzahl riechen ranzig und sind für Anwendungen unbrauchbar. Die Peroxidzahl gibt Aufschluß über den fortschreitenden Oxidationsprozeß bzw. die Menge der Lipidperoxide. Farb- und Geruchsveränderung sind weitere Merkmale für eine Destabilisierung der Pflanzenöle.

Die durch eine Destabilisierung der ungesättigten Triacylglycerine entstehenden Peroxide erhöhen das ungewünschte toxische Potential der Formulierungen. Zudem werden eine Reihe von kosmetisch erwünschten Effekten - durch die Destabilisierung - reduziert bis eliminiert: Beispielhaft sei dazu genannt:

Die mehrfach ungesättigten Fettsäuren, wie z.B. Linolsäure und gamma-Linolensäure bewirken eine erhöhte Hautelastizität durch Bildung von beweglichen und flexiblen Strukturen - mittels ihrer Ellenbogenkonfiguration (C-C-Doppelbindungen mit cis-Konfiguration) - in den Zellmembranen bzw. Hautlipiden. Durch einen hohen Anteil von mehrfach ungesättigten Fettsäuren wird eine höhere Packungsdichte der Hautlipide erreicht, dies führt zu einer Verstärkung der Barrierefunktion der Haut und dies wiederum zu einem verminderten transepidermalen Wasserverlust. Die Zellproliferation wird erhöht. Durch die Autoxidation wird die Zahl der C-C-Doppelbindungen und damit der für die Beweglichkeit und Flexibilität verantwortlichen Strukturen erniedrigt.

Neben der kosmetischen Anwendung von ungesättigte Triacylglycerine enthaltenden Ölen ist auch die topische oder orale Applikation als Arzneimittel oder als synthetisches Nahrungsmittel zu nennen. Bestimmte ungesättigte Fettsäuren, wie z.B. Linolsäure oder gamma-Linolensäure, sind für den Säugetierorganismus absolut notwendig, also essentiell, weil sie von ihm nicht synthetisiert werden können und deshalb extern zugeführt werden müssen.

Folgende Maßnahmen sind aus dem Stand der Technik bekannt, um Pflanzenöle, die mehrfach ungesättigte Fettsäuren enthalten, mittels Cyclodextrin zu stabilisieren:

In CA:107:22242k wird ein Cholesterin-senkendes Lebensmittel beschrieben, in welchem γ-Linolensäure mit α-Cyclodextrin formuliert wird.

Aus CA: 107:133049x sind Milch oder Milchpulver bekannt, die β-Cyclodextrin-Komplexevon Triacylglycerinen mit einem Anteil von 70% γ-Linolensäure enthalten.

In CA:87:116647s werden Cyclodextrin-Einschluß-Verbindungen von α- und β-Cyclodextrin mit Mono-, Di- und Triacylglycerinen am Beispiel Soyabohnenöl beschrieben.

In CA:108:220598 wird nach Lagerung eines Triacylglycerin-β-Cyclodextrin-Komplexes bei 40°C für einen Monat eine Reduktion der Peroxid-Bildung gegenüber nicht komplexiertem Triacylglycerin um über 50% gefunden.

Das Chemical Abstract CA:108:192767y beschreibt die Stabilisierung von γ-Linolensäure in Nachtkerzenöl durch den Einschluß in β-Cyclodextrin.

In CA:113:217812 wird β-Cyclodextrin zur Verbesserung der Emulsionseigenschaften von Fettsäuren in hautkosmetischen Formulierungen eingesetzt.

In dem Chemical Abstract CA: 107:46327t werden therapeutische Getränke beschrieben, die einen β-Cyclodextrin-Komplex von γ-Linolensäure enthalten.

In Journal of Inclusion Phenomena and Molecular Recognition in Chemistry 16 (1993), S. 339-354 wird die Stabilisierung von Leinsamenöl als α- und β-Cyclodextrin-Komplex gegenüber freiem Öl durch die Aufnahme von Sauerstoff in einer Warburg Apparatur nachgewiesen. Dort wird als besonders bevorzugt die Verwendung von α-Cyclodextrin offenbart. Auf S. 342, 3. Absatz von unten legen die Autoren nahe, daß die linearen Fettsäuren, sowohl frei als auch als Glycerinester, mit α-Cyclodextrin, dem Cyclodextrin mit der kleinsten Kavität, die stabilsten Komplexe bilden.

Die Aufgabe der Erfindung ist es, ein Verfahren zur Verfügung zu stellen, welches es ermöglicht, Pflanzenöle mit einem hohen Anteil an Triacylglycerinen enthaltend mehrfach ungesättigte Fettsäuren gegenüber oxidativer Zersetzung zu stabilisieren.

Eine weitere Aufgabe der Erfindung war es, ein Verfahren zur Verfügung zu stellen, welches es ermöglicht, Pflanzenöle in wäßrigen Medien zu dispergieren.

Die Aufgaben werden gelöst durch ein Verfahren, bei dem Cyclodextrin mit Pflanzenöl gemischt wird und so ein CD/Pflanzenöl Komplex gebildet wird, dadurch gekennzeichet, daß γ-Cyclodextrin zum Komplexieren der Pflanzenöle eingesetzt wird.

Mittels des Einsatzes von γ-Cyclodextrin läßt sich eine bessere Stabilisierung der Pflanzenöle erlangen als durch Einsatz von α- oder β-Cyclodextrin.

In keinem Dokument des Standes der Technik wird die Verwendung von γ-Cyclodextrin zur Komplexierung, Dispergierung oder Stabilisierung von Pflanzenölen beschrieben. Das Journal of Inclusion Phenomena and Molecular Recognition in Chemistry 16 (1993), S. 339-354 legt, wie bereits ausgeführt, nahe, für solche Zwecke α-Cyclodextrin zu verwenden. Diese Lehre der bevorzugten Verwendung des Cyclodextrins mit der kleinsten Kavität führt den Fachmann eher von der erfindungsgemäßen Lehre, nämlich der Verwendung des Cyclodextrins mit der größten Kavität weg.

Durch den Einschluß der Pflanzenöle in γ-CD wird eine ausgezeichnete Dispergierung der Pflanzenöle in Wasser oder wäßrigen Lösungen ermöglicht.

Pflanzenöle mit einem hohen Anteil an Triacylglycerinen enthaltend mehrfach ungesättigte Fettsäuren werden definiert durch den Gehalt an z.B. Linol- oder α- und γ- Linolensäure. Beispiele für solche Planzenöle sind: Weizenkeimöl, Borretschöl, Nachtkerzenöl, Schwarze-Johannisbeer-Öl, Leinöl, Sonnenblumenöl, Nußöle (Mandel, Erdnuß), Olivenöl.

Das erfindungsgemäße Verfahren eignet sich insbesondere zum Stabilisieren und Dispergieren von Pflanzenölen mit einem Gehalt an mehrfach ungesättigten Fettsäuren > 50%.

Die Zusammensetzung der Fettsäuren der Triacylglycerine läßt sich in bekannter Weise durch Analyse der entsprechenden Methylester durch Gaschromatographie bestimmen.

Die Pflanzenöle werden in an sich bekannter Art, beispielsweise durch Pressung, Destillation oder Extraktion mit einem organischen Lösungsmittel gewonnen. Typische Fettsäureprofile sind in folgender Tabelle ersichtlich:

**Tabelle 1:**

| Fettsäure | Nachtkerzenöl | Borretschöl | Schwarze-Johannisbeeröl |
|---|---|---|---|
| Palmitinsäure | 6 - 10 % | 9 - 13 % | 6 % |
| Stearinsäure | 1.5 - 3.5 % | 3 - 5 % | 1 % |
| Ölsäure | 6 - 12 % | 15 - 17 % | 10 - 12 % |
| Linolsäure | 74.2% | 40.4% | 48 % |
| Linolensäure | 8 - 12 % | 19 - 25 % | 30 % |
| andere | < 1 % | < 4 % | <3 % |

Es zeigte sich überraschend, daß sich Pflanzenöle hervorragend durch die Komplexierung mit γ-Cyclodextrin.stabilisieren und auch dispergieren lassen. Im Vergleich mit α- und β-Cyclodextrin wurde eine deutlich höhere Stabilisierung der ungesättigten Verbindungen gefunden. Die Peroxidzahlen der γ-CD-Formulierung lagen nach Lagerung an Luftsauerstoff (Beispiel 3) unter denen, die bei α- und β-CD erreicht wurden.

Die Erfindung betrifft somit auch Komplexe von Pflanzenölen mit einem hohen Anteil Triacylglycerin enthaltend mehrfach ungesättigte Fettsäuren mit γ-CD.

Durch die Komplexierung der Pflanzenöle mit γ-CD werden unerwartet stabile Dispersionen in wäßrigen Systemen erhalten. Die bevorzugte Teilchengröße der Komplexe beträgt ca. 10 - 100 µm.

Bei diesen Dispersionen ist das Öl-zu-Wasser Verhältnis vorzugsweise kleiner 1 (Öl-in-Wasser-Emulsion).

Die Erfindung betrifft somit auch Öl-in-Wasser-Emulsionen von Pflanzenöl/γ-CD Komplexen in wäßrigen Systemen.

Die Komplexe der Pflanzenöle mit γ-CD können in an sich bekannter Weise hergestellt werden. Dies kann z.B. aus Lösung, aus Suspension mit der Pastenmethode oder Knetmethode (Cyclodextrin Technology; J. Szejtli, Kluwer Academic Publishers, 1988, S. 87-90) geschehen.

Als vorteilhaft hat sich die Herstellung aus konzentrierten, wäßrigen γ-CD-Lösungen erwiesen. Dazu wird das Pflanzenöl der wäßrigen γ-CD Lösung zugesetzt. Die CD-Konzentration der wäßrigen Lösung (vor Zusatz von Pflanzenöl) liegt vorzugsweise zwischen 5 - 50 Gewichts-%. Besonders bevorzugt ist eine CD-Konzentration von 20- 50 Gew.%.

Das Gewichts-Verhältnis Pflanzenöl zu CD liegt vorzugsweise zwischen 1 : 20 und 1 : 0,3, besonders bevorzugt zwischen 1 : 10 und 1 : 0,5.

Pflanzenöl und γ-CD/γ-CD-Lösung werden portionsweise oder kontinuierlich vermischt.

Die Ansätze werden intensiv vermischt, d.h. je nach Konsistenz intensiv gerührt oder geknetet.

Dies geschieht vorzugsweise in einem Temperaturbereich von oberhalb des Gefrierpunktes bis 80°C. Besonders bevorzugt wird bei 20 - 60 °C, insbesondere bei etwa 30 - 50 °C gearbeitet. Die Mischdauer hängt von der Temperatur ab und liegt vorzugsweise zwischen einer Stunde und einigen Tagen. In der Regel ist eine Mischzeit von 10 bis 30 Stunden ausreichend.

Die Komplexierung erfolgt vorzugsweise unter Normaldruck.

Bevorzugt findet die Komplexierung unter Schutzgasatmosphäre (Stickstoff oder Argon) statt.

Die schlecht wasserlöslichen Komplexe können direkt in Form der Reaktionsmischung verwendet werden. Sie können aber auch durch Filtration, Zentrifugation, Trocknung, Mahlen, Sieben, Sichten, Granulieren, Tablettieren entsprechend der jeweils üblichen Vorgehensweise isoliert und aufbereitet werden.

Je nach Einsatzzweck z.B. in kosmetischen Formulierungen können noch weitere Substanzen den γ-Cyclodextrin-Komplexen zugesetzt werden. So können z.B. Tenside, waschaktive, pflegende, selbstbräunende Zusätze, Verdickungsmittel, Konservierungsmittel, Stabilisatoren, Emulgatoren, Duftstoffe, Farbstoffe, Antioxidantien, Vitamine, UV-Filter, Silikonöle zugesetzt werden. Das Zusetzen der Substanzen kann während des Komplexierens oder danach erfolgen.

Vorzugsweise erfolgt das Zusetzen im Anschluß an das Komplexieren.

Der Einsatz der erfindungsgemäßen Komplexe von Pflanzenölen mit Triacylglycerinen, die mehrfach ungesättigte Fettsäuren enthalten, führt zu homogenen kosmetischen bzw. pharmazeutischen Zubereitungen vom Typ der O/W-Emulsionen, die über lange Zeit lagerstabil sind, sich nicht entmischen und eine vorteilhaft hohe und dabei konstante Viskosität aufweisen.

Die erfindungsgemäßen Komplexe bzw. Dispersionen lassen sich beispielsweise in kosmetischen Zubereitungen von Badepräparaten (Salz, Dusch- u. Schaumbäder), kosmetischen Dispersionen (Cremes, Masken, Emulsionen, Puder, Deodorante), Dekorativer Kosmetik (Make-up, Puder, Lippenstiftmassen), Sonnenschutzpräparaten, Haarpflegemitteln (Shampoo, Spülung, Packung), Repellentien oder Seifen verwenden.

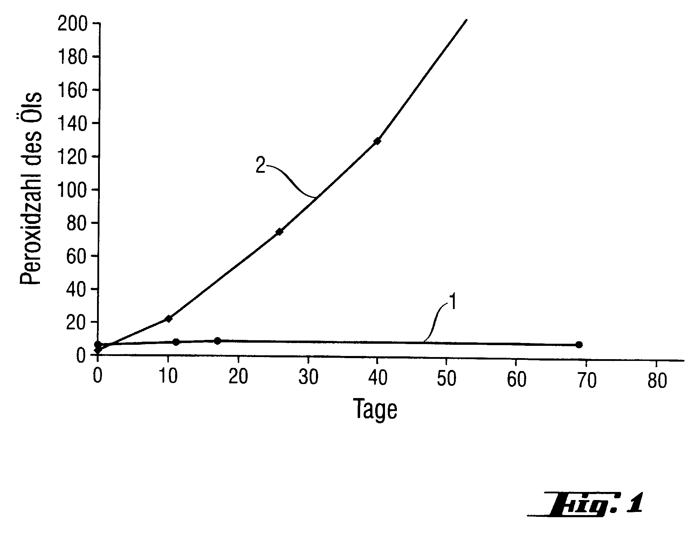

Fig.1 zeigt in Kurve 1 die Peroxidzahl des γ-Cyclodextrin.Komplexes aus Bsp. 5a. In Kurve 2 wird die Zunahme der Peroxidzahl der Stärkeverreibung nach Bsp. 5b dargestellt.

Die folgenden Beispiele dienen der weiteren Erläuterung der Erfindung.

### Beispiel 1: Komplexierung von Nachtkerzenöl mit α-, β-, γ-CD

a) 69,5 g α-CD wurden mit 162 ml dest. Wasser verrührt, auf 95°C aufgeheizt und unter Stickstoff auf 45°C abgekühlt. Bei dieser Temperatur wurden 20.0 g Nachtkerzenöl (POZ = 2,9) zugegeben und der Ansatz 24h gerührt. Nach dem Abkühlen auf Raumtemperatur wurde der entstandene Komplex abgesaugt und im Vakuum getrocknet.
b) 81,1 g β-CD wurden mit 189 ml dest. Wasser verrührt, auf 95°C aufgeheizt und unter Stickstoff auf 45°C abgekühlt. Bei dieser Temperatur wurden 20,0 g Nachtkerzenöl (POZ = 2,9) zugegeben und der Ansatz 24h gerührt. Nach dem Abkühlen auf Raumtemperatur wurde der Komplex durch Gefriertrocknung isoliert.
c) 833.8 g γ-CD wurden in einem thermostatisierten Planschliffgefäß mit 1945 ml dest. Wasser verrührt, auf 90°C aufgeheizt und unter Stickstoff auf 45°C abgekühlt. Bei dieser Temperatur wurden 180.0 g Nachtkerzenöl (POZ = 2.9) zugegeben und der Ansatz 30h gerührt. Nach dem Abkühlen auf Raumtemperatur wurde der entstandene Komplex abgesaugt und im Vakuum getrocknet.

Tabelle 2 zeigt die Zusammensetzung der Komplexe gemäß Beispiel 1.

**Tab. 2:**

| Komplex | Ausb. [g] | Ölgehalt [%] | POZ direkt nach Komplexierung | Trockenverlust [%] |
|---|---|---|---|---|
| 1a) | 40.5 | 36.3 | 3.1 | 3.4 |
| 1b) | 102.7 | 20.0 | 4.2 | 11.2 |
| 1c) | 802.1 | 22.6 | 3.7 | 10.5 |

### Beispiel 2: Bestimmung der Peroxidzahl des in Cyclodextrine eingeschlossenen Öls

Zur Dekomplexierung wurden je 5 g-10 g Nachtkerzenöl-Cyclodextrin-Komplex (aus Bsp 1a-c) in einem Gemisch aus 90 ml Methanol und 60 ml Petrolether eine Stunde bei Raumtemperatur gerührt. Das ungelöste Cyclodextrin wurde durch Filtration abgetrennt und das freie Öl durch Abdestillation des Lösungsmittels im Vakuum bei max. 30°C isoliert. Mit dem so erhaltenen Öl wurde eine Bestimmung der Peroxidzahl nach DAB 10 durch iodometrische Titration durchgeführt.

### Beispiel 3: Bestimmung der Lagerstabilität von Nachtkerzenöl als α-, β-,γ-CD-Komplex

Jeweils 50 g der Komplexe (nach Beispiel 1a-c) von Nachtkerzenöl mit α-, β- ,γ-CD wurden in flache Petrischalen gefüllt und bei Raumtemperatur bei Tageslicht (Fensterbank) gelagert. Durch Umrühren wurden die Proben vor Messung der Peroxidzahlen (analog Beispiel 2) des eingeschlossenen Öls homogenisiert, da an der Oberfläche eine verstärkte Oxidation (Gelbfärbung) stattfindet. In Tabelle 3 und 4 sind die Peroxidzahlen, Aussehen und Geruch nach verschiedenen Lagerzeiten aufgeführt. Der stabilisierende Effekt, erkennbar an der niedrigen Peroxidzahl, am geringen Geruch und der fehlenden Färbung ist beim γ-CD-Komplex am stärksten ausgeprägt.

**Tab. 3:**

| Lagerung 22 Tage | | | |
|---|---|---|---|
| Komplex | POZ | Aussehen | Geruch |
| 1a) | 115 | fast weiß | leicht ranzig |
| 1b) | 209 | gelb | deutlich ranzig |
| 1c) | 83 | weiß | geruchsneutral |

**Tab. 4:**

| Lagerung 38 Tage | | | |
|---|---|---|---|
| Komplex | POZ | Aussehen | Geruch |
| 1a) | 159 | gelblich | ranzig |
| 1b) | 251 | stark gelb | stark ranzig |
| 1c) | 113 | weiß | fast geruchsneutral |

### Beispiel 4: Komplexierung von Weizenkeimöl mit γ-CD

657.9 g trockenes γ-CD wurden bei 40°C in 3000 ml dest. Wasser gelöst. Anschließend wurden innerhalb von 50 min. 355 g Weizenkeimöl portionsweise zugegeben. Der Ansatz wurde 8h bei 40°C und 64h bei 25°C weitergerührt. Danach wurde der entstandene Komplex abfiltriert und das Produkt bei 35°C im Vakuum getrocknet.

### Beispiel 5: Vergleich eines γ-CD-Komplexes mit einer Stärkeverreibung von Nachtkerzenöl

- 5a:: 100 g γ-CD wurden bei 50°C in 200 ml dest. Wasser gelöst. Nach Zugabe von 5 g Nachtkerzenöl wurde die Reaktionsmischung 16 h gerührt und die Mischung gefriergetrocknet. Ausbeute: 102 g mit einem Ölgehalt von 5%.
- 5b:: 100 g Kartoffelstärke wurden in einer Reibschale intensiv mit 5 g Nachtkerzenöl verrieben, bis ein homogenes Pulver mit einem Ölgehalt von ca. 5% erhalten wurde.

Je 50 g des γ-CD-Komplexes (5a) und der Stärkeverreibung (5b) wurden in Petrischalen gefüllt und in einem Trockenschrank bei 37°C gelagert. In zeitlichen Abständen von wenigen Tagen bis Wochen wurden Proben von ca. 10 g der Substanzen 5a und 5b gezogen und die Peroxidzahl des Nachtkerzenöls, wie in Beispiel 2 beschrieben, bestimmt.

Wie in Fig. 1 ersichtlich, ist beim Einschluß in γ-Cyclodextrin eine sehr gute Stabilisierung des Öls über einen Zeitraum von mehreren Wochen erreicht worden, während im Vergleich bei der Stärkeverreibung die Autoxidation des Öls mit zunehmender Zeit voranschreitet.

### Beispiel 6: Komplexierung von Borretschöl mit γ-CD

Eine Lösung von 80.0 g γ-CD in 200 ml dest. Wasser wurde bei 40°C innerhalb von 2-3 min. mit 17.3 g Borretschöl versetzt. Nach innigem Vermengen mit einem Dispergiergerät (Ultra-Turrax) für 30 Minuten wurde die Mischung 24h bei 40°C und danach 12 h bei Raumtemperatur gerührt und der ausgefallene Komplex abfiltriert. Die Ausbeute betrug 81.7 g mit einem Feuchtegehalt von 6.5%. Der Ölgehalt belief sich auf 19.4%, bezogen auf Trockensubstanz.

### Beispiel 7: Komplexierung von Schwarzem Johannisbeeröl mit γ-CD

104.3 g γ-CD wurden unter Erhitzen auf 95°C in 130 ml dest. Wasser gelöst und unter Stickstoffzufuhr auf 40°C abgekühlt.

Bei dieser Temperatur wurden 22.5 g Schwarzes Johannisbeeröl zugegeben und der pastöse Ansatz 20 h in einem Planetenmischer geknetet. Die Paste wurde in einem Vakuumtrockenofen bei 35°C bei einem Druck von 1- 3 mm Hg getrocknet. Der getrocknete Komplex wurde anschließend in einem Labormixer zerkleinert und mit einem Sieb der Maschenweite 200 µm gesiebt. Die Ausbeute betrug 118 g mit einer POZ von 7.4. Nach zweiwöchiger Lagerung bei Raumtemperatur konnte noch kein ranziger Geruch festgestellt werden.

### Beispiel 8: Ölschaumbad mit Weizenkeimöl

| | |
|---|---|
| Wasser | 53g |
| γ-Cyclodextrin | 16g |
| Weizenkeimöl | 6g |
| Kokosfettalkoholethersulfat | 23g |
| Methylparaben | 0,1g |
| Parfümöl | 1,9 g |

### Herstellung:

In die wäßrige Lösung von γ-Cyclodextrin wird unter N₂-Spülung bei 30°C das Weizenkeimöl eingerührt. Nach einer Rührzeit von 3h werden der Reihe nach die weiteren Komponenten zugemischt und 2h weitergerührt.

### Beispiel 9: Feuchtigkeitslotion mit Nachtkerzenöl

| | |
|---|---|
| Wasser | 62g |
| γ-Cyclodextrin | 14g |
| Nachtkerzenöl | 3g |
| Siloxane Polyglycoside | 13g |
| Isooctadecyl Isononanoate | 2g |
| Vaseline | 2g |
| Laureth | 3g |
| Methylparaben | 0,1g |
| Parfümöl | 0,9g |

### Herstellung:

In die wäßrige Lösung von γ-Cyclodextrin wird unter N₂-Spülung bei Raumtemperatur das Nachtkerzenöl eingerührt. Nach einer Rührzeit von 5h werden der Reihe nach die weiteren Komponenten zugemischt und 2h weitergerührt.

### Beispiel 10: Haar-Shampoo mit pflegender, konditionierender Eigenschaft

| | |
|---|---|
| Wasser | 52g |
| γ-Cyclodextrin | 8g |
| Schwarzes Johannisbeeröl | 2g |
| Natriumlaurylsulfat | 19g |
| Cocoamidopropyl-Betain | 10g |
| Dimethicone DM 350 | 2g |
| Cocamide MEA | 6g |
| Parfümöl | 1g |

### Herstellung:

In die wäßrige Lösung von gamma-Cyclodextrin wird unter N₂-Spülung bei Raumtemperatur das Johannisbeeröl eingerührt. Nach einer Rührzeit von 3h werden der Reihe nach die weiteren Komponenten zugemischt und 80 min weitergerührt.

### Beispiel 11: Badesalz, rückfettend, pflegend

| | |
|---|---|
| Natriumlaurylsulfat | 20g |
| Natriumsesquicarbonat | 40g |
| γ-Cyclodextrin-Komplex mit Nachtkerzenöl nach Beispiel 1c | 40g |

Die genannten Komponenten werden in einer Kugelmühle 30 Minuten homogenisiert.

### Beispiel 12: Komplexierung von Sonnenblumenöl mit α-CD

In einem 1000 ml Glas-Kolben werden 120 g α-Cyclodextrin in 650 ml Wasser gelöst und 220 g Sonnenblumenöl zugegeben. Mit einem KPG-Rührer wird die Mischung bei 40°C für 24 Stunden gerührt. Es entsteht eine sehr feine Dispersion des Öls in Wasser.
Beobachtung: Die Viskosität (Brookfield-Viskosimeter: 10 upm; Spindel S2 , Viskosität 310mPas) der Emulsion bleibt bei Lagerung bei 25°C über 5 Tage gleich.
Nach ca. 14 Tagen ist eine Separation der Phasen zu beobachten.

### Beispiel 13: Komplexierung von Sonnenblumenöl mit β-CD

Ausführung wie Beispiel 12, nur mit 120 g β-Cyclodextrin. Beobachtung: Zu Beginn entsteht eine feine Dispersion Öl-in-Wasser. Die Viskosität (Brookfield, wie 1a) nimmt im Laufe von 14 Tagen nur leicht von 100mPas auf 500 mPas zu.

### Beispiel 14: Komplexierung von Sonnenblumenöl mit γ-CD

Ausführung wie Beispiel 12, nur mit 120 g γ-Cyclodextrin. Beobachtung: Es entsteht eine stabile Öl-in-Wasser Emulsion. Die Viskosität (Anfangswert: 350 mPas) steigt im Verlauf von 14 Tagen bis zu einer cremeartigen Konsistenz stark an (Viskosität: 25000 mPas). Auch nach 4 Wochen wird keine Phasenseparation beobachtet.

## Patentansprüche

1. Verfahren zum Dispergieren von Pflanzenölen mit einem hohen Anteil an Triacylglycerinen enthaltend mehrfach ungesättigte Fettsäuren in einem wäßrigen Medium, dadurch gekennzeichet, daß das Pflanzenöl in Form eines γ-Cyclodextrin/Pflanzenöl Komplexes eigesetzt wird.

2. Verfahren gemäß Anspruch 1, dadurch gekennzeichnet, daß das Gewichts-Verhältnis Pflanzenöl zu CD zwischen 1 : 10 und 1 : 0,5 liegt.

3. Verfahren gemäß einem der Ansprüche 1 oder 2, dadurch gekennzeichnet, daß die Komplexbildung im Temperaturbereich von oberhalb des Gefrierpunktes bis 80°C durchgeführt wird.

4. Verfahren gemäß einem der Ansprüche 1 bis 3, dadurch gekennzeichnet, daß die Mischdauer zwischen einer Stunde und einigen Tagen liegt.

5. Verfahren gemäß einem der Ansprüche 1 bis 4, dadurch gekennzeichnet, daß die Komplexierung unter Schutzgasatmosphäre (Stickstoff oder Argon) stattfindet.

## Claims

1. Method of dispersing vegetable oils with a high proportion of triacylglycerols comprising polyunsaturated fatty acids in an aqueous medium, characterized in that the vegetable oil is employed in the form of a γ-cyclodextrin/vegetable oil complex.

2. Method according to Claim 1, characterized in that the weight ratio of vegetable oil to CD is between 1 : 10 and 1 : 0.5.

3. Method according to either of Claims 1 or 2, characterized in that complex formation is effected in a temperature range from above freezing point to 80°C.

4. Method according to any of Claims 1 to 3, characterized in that the mixing time is between one hour and several days.

5. Method according to any of Claims 1 to 4, characterized in that complexation is effected under protective gas atmosphere (nitrogen or argon).

## Revendications

1. Procédé de dispersion d'huiles végétales avec une proportion élevée de triacylglycérols contenant des acides gras polyinsaturés dans un milieu aqueux, caractérisé en ce que l'huile végétale est utilisée sous forme d'un complexe de γ-cyclodextrine/huile végétale.

2. Procédé suivant la revendication 1, caractérisé en ce que le rapport pondéral huile végétale à CD se situe entre 1:10 et 1:0,5.

3. Procédé suivant l'une des revendications 1 ou 2, caractérisé en ce que la formation de complexe est effectuée dans l'intervalle de température d'au-delà du point de congélation jusqu'à 80°C.

4. Procédé suivant l'une des revendications 1 à 3, caractérisé en ce que la durée de mélange se situe entre une heure et quelques jours.

5. Procédé suivant l'une des revendications 1 à 4, caractérisé en ce que la complexation a lieu sous atmosphère de gaz protecteur (azote ou argon).
